# EUROPÄISCHE PATENTANMELDUNG

(11) **EP 3 006 107 A1**
(43) Veröffentlichungstag der Anmeldung: **13.04.2016**
(21) Anmeldenummer: 15187914.5
(22) Anmeldetag: 01.10.2015
(51) Int. Cl.: B01J 35/00, B01J 21/06

(54) **KATALYSATORSYSTEM ZUR HERSTELLUNG VON KETONEN AUS EPOXIDEN**

(30) Priorität: 02.10.2014 EP 14187482
(71) Anmelder: Evonik Degussa GmbH, 45128 Essen (DE)
(72) Erfinder: Micoine, Kévin, 45701 Herten (DE); Meier, Ralf, 44265 Dortmund (DE); Herwig, Jürgen, 46569 Hünxe (DE); Bétard, Angélique, 45701 Herten (DE); Quandt, Thomas, 45772 Marl (DE)

(57) **Zusammenfassung**

Es wird eine Katalysatorzusammensetzung zur Verfügung gestellt, die mindestens ein Edelmetall und mindestens ein Mischoxid umfassend Zirconiumdioxid und Siliciumdioxid umfasst. Das Edelmetall ist geträgert und der Träger besteht nicht aus dem Mischoxid. Das Massenverhältnis von Zirconiumdioxid zu Siliciumdioxid im Mischoxid beträgt 86 : 14 bis 99,9 : 0,1.

Das Katalysatorsystem kann zur Herstellung eines Ketons aus einer mindestens eine Epoxidgruppe enthaltenden Verbindung verwendet werden

## Beschreibung

Die vorliegende Erfindung betrifft eine Katalysatorzusammensetzung umfassend mindestens ein Edelmetall und mindestens ein Mischoxid enthaltend Zirconiumdioxid und Siliciumdioxid. Weiterhin bildet ihre Herstellung und ihre Verwendung einen Gegenstand der Erfindung. Darüber hinaus werden ein Verfahren zur Herstellung von Ketonen und ein Verfahren zur Synthese von Lactamen beansprucht.

In der Patentanmeldung EP-A-2772478 (US 2014/0249331) wurde ein Verfahren zur Herstellung eines Ketons aus einem Epoxid unter Einsatz eines Gemisches umfassend mindestens ein Edelmetall und mindestens ein Metalloxid als Katalysatorsystem beschrieben, wobei das Metalloxid Zirconiumdioxid enthält. Mit reinem Zirconiumdioxid als Metalloxid konnte das Epoxycyclododecan mit Ausbeuten bis über 96 % in Cyclododecanon umgesetzt werden.

Obwohl dieses Verfahren das Keton in hoher Selektivität bereitstellt, sind erhebliche Katalysatormengen, lange Reaktionszeiten und hohe Temperaturen erforderlich, was ein etwaiges industrielles Verfahren wirtschaftlich negativ beeinflusst.

Es bestand nunmehr die Aufgabe, die Umlagerung von Epoxiden in Ketone derart anzupassen, dass die Katalysatormenge oder die Reaktionszeit reduziert werden können. Zudem sollte das Keton in zumindest gleichhoher Selektivität und/oder Ausbeute gewonnen werden. Es wäre außerdem ein verfahrenstechnischer Vorteil, wenn die Temperatur der Reaktion geringer ausfallen könnte als in Verfahren des Standes der Technik.

Es wurden nun Katalysatorzusammensetzungen der eingangs genannten Art gefunden, die die Aufgabe lösen können.

Die Zusammensetzungen umfassen mindestens ein Edelmetall und mindestens ein Mischoxid, wobei das Mischoxid Zirconiumdioxid und Silicumdioxid enthält. Das Massenverhältnis von Zirconiumdioxid zu Siliciumdioxid im Mischoxid beträgt 86 : 14 bis 99,9 : 0,1, vorzugsweise 90 : 10 bis 97 : 3. Durch das angegebene Massenverhältnis ist beispielsweise Zirconiumdioxid-dotiertes Siliciumdioxid ausgeschlossen. Das Massenverhältnis wird auf Basis der für das Mischoxid eingesetzten Zirconium- bzw. Siliciumverbindungen berechnet.

In einer ersten Ausführungsform ist das Edelmetall nicht geträgert (System I).

In einer zweiten Ausführungsform ist das Edelmetall geträgert, wobei der Träger nicht aus dem Mischoxid besteht (System II). Der Begriff "besteht nicht" schließt damit abschließend einen Träger aus, der 100 Gew.-% Mischoxid, bezogen auf das Gesamtgewicht des Trägers, beinhaltet. In einer bevorzugten Ausführungsform enthält der Träger nicht das Mischoxid oder besteht daraus. Diese bevorzugte Ausführungsform umfasst somit sowohl den Fall, dass der Träger nicht aus dem Mischoxid besteht, als auch den Fall, dass der Träger das Mischoxid nicht enthält. Der letztgenannte Fall umfasst den Grenzfall, dass der Träger nicht aus dem Mischoxid besteht. Ausgenommen sind technisch bedingte Verunreinigungen bis zu 1 Gew.-% Mischoxid, bezogen auf das Gesamtgewicht des Trägers.

Der Träger des Edelmetalls ist vorzugsweise ausgewählt aus Siliciumdioxid, Aluminiumoxid, Aktivkohle oder Mischungen daraus, wobei Siliciumdioxid bevorzugt ist. Die spezifische Oberfläche des Mischoxids, nach BET-Verfahren gemessen, beträgt vorzugsweise 5 - 155 m²/g.

In einer dritten Ausführungsform der Erfindung (System III) kann das Mischoxid nicht aus Organosiliciumverbindungen herstellbar sein. Die spezifische Oberfläche des Mischoxids, nach BET-Verfahren gemessen, beträgt 5 - 155 m²/g und das Edelmetall ist auf dem Mischoxid geträgert (das Mischoxid fungiert als Träger). Hierbei weist das Mischoxid eine monomodale Porenradienverteilung auf. Hierbei ist es bevorzugt, dass das Mischoxid aus Siliciumdioxid herstellbar ist, das eine Korngröße von mindestens 100 nm aufweist.

Die Porenradienverteilung kann beispielsweise durch die Zugabe von Porenbildnern beeinflusst werden. Hierbei kann der Fachmann durch entsprechende Vorversuche ermitteln, ob monomodale oder höhergradige Verteilungen erhalten werden.

Die Porenradienverteilung der Mischoxide wird mittels Quecksilberporosimetrie ermittelt. Quecksilberporosimetrie wurde auf Pascal 440 und Pascal 140 Geräten von CE Instruments mit einem maximalen Druck von 4000 bar gemäß DIN 66133 gemessen.

Die drei Ausführungsformen der Erfindung umfassend die Systeme I, II und III werden als erfindungsgemäße Katalysatorsysteme bezeichnet.

Die Systeme II und III sind bevorzugte Katalysatorzusammensetzungen, wobei System II besonders bevorzugt ist. Der Vorteil des Systems II liegt darin, dass Träger und Edelmetall, insbesondere bei kontinuierlichen Verfahren, getrennt vom Mischoxid vorliegen können. Hierdurch wird eine getrennte Reaktionsführung ermöglicht. Darüber hinaus verringert die Trennung des Edelmetalls vom Mischoxid die Alterung des Edelmetalls (mit der Alterung verringert sich die Selektivität).

Das Mischoxid der Systeme I und II weist vorzugsweise eine BET-Oberfläche auf, die im Bereich von 5 - 155 m²/g liegt.

Es ist bevorzugt, dass die BET-Oberfläche der Mischoxide der erfindungsgemäßen Katalysatorsysteme in einem Bereich von 80 bis 150 m²/g liegt. Die BET-Oberfläche wird gemäß DIN 66131 und DIN ISO 9277 gemessen. Eine BET-Oberfläche oberhalb von 155 m²/g führt zu einer geringeren Selektivität.

Überraschenderweise katalysieren die erfindungsgemäßen Katalysatorzusammensetzungen bei der Umlagerung von Epoxidverbindungen die Bildung der Ketone. Das Keton kann in hoher Ausbeute und Reinheit gewonnen werden. Weiterhin können gegenüber dem Stand der Technik geringere Katalysatormengen eingesetzt und/oder kürzere Reaktionszeiten realisiert werden. Darüber hinaus werden die Reaktionen bei geringeren Temperaturen katalysiert.

Katalysatorzusammensetzungen mit Mischoxiden außerhalb des genannten Massenverhältnisses zeigen deutlich geringere Aktivitäten. Darüber hinaus nimmt die Selektivität der Ketonbildung bei der Umlagerung von Epoxiden ab.

Unter Mischoxid wird im Sinne der vorliegenden Erfindung eine Zusammensetzung verstanden, die zumindest Zirconiumdioxid und Siliciumdioxid umfasst. Zirconiumdioxid und Siliciumdioxid liegen im Mischoxid nicht als konkrete Verbindungen vor, sondern dienen lediglich als Grundlage zur Berechnung der Massenverhältnisse. Das Mischoxid wird durch Calcination erhalten. Das Mischoxid stellt somit keine physikalische Mischung von Zirconiumdioxid und Siliciumdioxid dar, sondern eine chemische Mischung enthaltend mindestens Silicium- und Zirconium-Kationen mit einer eigenen Kristallstruktur. Insofern stellt eine physikalische Mischung, welche zumindest die beiden Oxide umfasst und nicht calciniert wurde, kein Mischoxid im Sinne der Erfindung dar. Ebenso wenig ist Zirconiumdioxid-dotiertes oder beschichtetes Siliciumdioxid umfasst.

Das Mischoxid umfasst Zirconiumdioxid und Siliciumdioxid oder besteht aus diesen beiden Oxiden. Der Anteil der Summe aus Zirconiumdioxid und Siliciumdioxid im Mischoxid beträgt vorzugsweise mindestens 20 Gew.-%, und bevorzugt mindestens 30 Gew.-%, besonders bevorzugt 50 Gew.-% und ganz besonders bevorzugt 95 Gew.-%, jeweils bezogen auf das Gesamtgewicht des Mischoxids. Besonders bevorzugt besteht das Mischoxid aus Zirconiumdioxid und Siliciumdioxid.

Das Mischoxid des Katalysatorsystems kann eine durchschnittliche Schüttdichte von 0,5 bis 2 g/cm³ aufweisen. Die Schüttdichte wird gemessen, indem zunächst ein leerer 1000-mL-Meßzylinder gewogen wird. Anschließend wird das Mischoxid bis zur 500-mL-Marke eingefüllt. Der gefüllte Zylinder wird erneut gewogen, aus der Gewichtsdifferenz von gefülltem und leerem Meßzylinder wird die Schüttdichte des Materials in g/cm³ angegeben.

Die erfindungsgemäßen Katalysatorzusammensetzungen umfassen das Mischoxid. Darüber hinaus kann ein Träger für das Edelmetall vorhanden sein, der nicht aus dem Mischoxid besteht (inerter Träger, System II). Vorzugsweise enthält der Träger nicht das Mischoxid oder besteht daraus. Das Mischoxid als Träger sowie der inerter Träger können als Pulver oder als Formkörper vorliegen, wobei die Träger als Formkörper bevorzugt sind. Ebenso ist es bevorzugt, dass das Mischoxid als Formkörper vorliegt, wenn das Mischoxid nicht als Edelmetall-Träger fungiert.

Geeignete Formkörper sind Kugeln, Extrudate, Tabletten, Granulate und Pellets. Die Umsetzung von Pulver in Formkörper ist zum Beispiel im Kapitel 9 "Shaping of Solid Catalysts" des Buches "Synthesis of Solid Catalysts", ed K. P. de Jong, Wiley-VCH Verlag GmbH & Co. KGaA, Weinheim, Germany (2009) beschrieben.

Das gewichtsbezogene Verhältnis von Mischoxid zu Edelmetall kann bei allen Katalysatorsystemen 99,9 : 0,1 bis 50 : 50, vorzugsweise 99,5 : 0,5 bis 80 : 20 und bevorzugt 99:1 bis 90:10 betragen.

Wenn der Edelmetall geträgert ist (Katalysatorsysteme II und III), kann der Anteil an Edelmetall, bezogen auf das Gesamtgewicht aus Edelmetall und Träger, 0,01 bis 5 Gew.-%, vorzugsweise 0,05 bis 2,5 Gew.-% und bevorzugt 0,3 bis 2,0 Gew.-% betragen. Das Edelmetall kann auf oder im Träger verteilt sein.

Das Edelmetall des Katalysatorsystems wird vorzugsweise ausgewählt aus Ruthenium, Rhodium, Palladium, Osmium, Iridium, Platin und Mischungen davon, wobei Ruthenium, Palladium und Platin bevorzugt und Ruthenium und Palladium besonders bevorzugt sind und Palladium ganz besonders bevorzugt ist. Platin ist in der Reihe der drei Edelmetalle Ruthenium, Palladium und Platin wegen seiner geringeren Selektivität gegenüber CDON weniger geeignet. Das Edelmetall kann als Pulver (ungeträgert) oder geträgert vorliegen. Die Edelmetalle können als elementare Metalle oder in Form ihrer Oxide vorliegen, wobei elementare Metalle bevorzugt sind.

Ein weiterer Gegenstand der Erfindung ist ein Verfahren zur Herstellung der erfindungsgemäßen Katalysatorzusammensetzung. Die Zusammensetzung umfasst mindestens ein Edelmetall und mindestens ein Mischoxid. Zur Herstellung des Mischoxids wird zunächst eine formbare Masse hergestellt, welche zumindest eine Zirconiumverbindung, Siliciumdioxid als Feststoff und Wasser umfasst. Die formbare Masse wird anschließend bei einer Temperatur von 300 bis 500 °C, vorzugsweise 400 bis 500 °C, calciniert. Zur Herstellung des Systems III ist es notwendig, dass das Siliciumdioxid eine Korngröße d₅₀ von mindestens 100 nm aufweist. Für das System III ist es bevorzugt, dass keine Polymere, ausgewählt aus Polyalkylenpyrrolidonen wie Polyvinylpyrrolidon, Polyaminen, Polyacrylaten, Polyalkoholen, Polysiloxanen oder deren Mischungen, zugesetzt werden. Hierdurch wird verhindert, dass eine bimodale Porenradienverteilung entsteht.

Unter einem Siliciumdioxid als Feststoff wird ein Pulver verstanden, das keine stabile Dispersion in Wasser bildet. Das Siliciumdioxid als Feststoff weist vorzugsweise eine Korngröße d₅₀ kleiner als 500 µm auf. Bevorzugt ist die Korngröße kleiner als 500µm, wobei diese Obergrenze mittels Siebanalyse ermittelt wird (Maschenweite von 500 µm; diese Obergrenze, mittels Siebanalyse bestimmt, ist folglich ein Maximalwert und kein Mittelwert d₅₀). Ebenso weist der Feststoff vorzugsweise eine Korngröße d₅₀ größer als 100 nm (für Systeme I und II). Für alle Systeme I, II und III ist die Korngröße d₅₀ bevorzugt größer als 500 nm und besonders bevorzugt größer als 1 µm auf. Die Partikelgröße d₅₀ wird durch Laserbeugung nach ISO 13320:2009 ermittelt werden. Für den Messvorgang wird Feststoff (gemäß Herstellerangabe) in der Dispergiereinheit Scirocco 2000 eines Malvern Mastersizer 2000 vorgelegt. Der Druck wird bei 1 bar gewählt und die Messung durchgeführt.

Kolloidale Lösungen von SiO₂ wie Ludox (Grace Davison) und Köstrosol (CWK Bad Köstritz) sind nicht bevorzugt. Derartige kolloidale Lösungen enthalten in der Regel einzelne SiO₂-Partikel mit einer Größe d₅₀ zwischen 5 und 30 nm. Um die Partikelgröße von SiO₂ in einer kolloidalen Lösung zu ermitteln, wird ein Zetasizer der Nano Linie von Malvern eingesetzt. Die Messung wird bei Raumtemperatur durchgeführt.

Vorzugsweise wird das Siliciumdioxid mittels Pyrogenverfahren hergestellt. Das Verfahren ist dem Fachmann bekannt, z.B. aus der Schriftenreihe "Fine Particles" Nr. 11 (7. Ausgabe, 2003), Firmenzeitschrift der Degussa AG.

Die Zirconiumverbindung ist vorzugsweise ausgewählt aus Zirconiumdioxid, Zirconiumhydroxid, Zirconiumacetat, Zirconiumnitrat, Zirconiumoxychlorid, Ammoniumzirconiumcarbonat oder Mischungen daraus. Bevorzugt werden Zirconiumdioxid, Zirconiumhydroxid oder Mischungen daraus eingesetzt.

Als Zirconiumhydroxid wird Zirconium-(IV)-hydroxid verstanden.

Es ist ebenfalls bevorzugt, die Zirconiumverbindung aus einer Mischung umfassend A und B auszuwählen. Hierbei umfasst A Zirconiumdioxid, Zirconiumhydroxid und Mischungen daraus. B ist ausgewählt aus Zirconiumacetat, Zirconiumnitrat, Zirconiumoxychlorid, Ammoniumzirconiumcarbonat und Mischungen daraus. Der Anteil an Zirconium aus A beträgt vorzugsweise mindestens 85 mol-%, bevorzugt mindestens 90 mol-%, bezogen auf die Summe an Zirconium aus A und B.

Durch die Calcinierung werden die Zirconiumverbindungen zumindest teilweise zu Zirconiumdioxid umgesetzt. Insofern werden Zirconiumverbindungen, außer Zirconiumdioxid selbst, als Präkursoren bezeichnet.

Vorzugsweise wählt der Fachmann zeitliche Bedingungen, durch die mindestens 50 mol-%, bevorzugt mindestens 90 mol-%, besonders bevorzugt 95 mol-% und ganz besonders bevorzugt 100 mol-% der Zirconiumverbindungen, jeweils bezogen auf die Summe aller Zirconiumverbindungen, zu Zirconiumdioxid reagiert haben.

Zur Herstellung des Systems II kann das Edelmetall auf einen inerten Träger imprägniert werden, wobei der Träger nicht aus dem Mischoxid besteht. Vorzugsweise enthält der Träger nicht das Mischoxid oder besteht daraus. Zu diesem Zweck kann jedes dem Fachmann bekannte Imprägnierungsverfahren wie der Auftrag einer Edelmetalllösung auf den Träger eingesetzt werden.

Zur Herstellung des Systems III kann das Edelmetall auf dem Mischoxid als Träger imprägniert werden. Zu diesem Zweck kann jedes dem Fachmann bekannte Imprägnierungsverfahren wie der Auftrag einer Edelmetalllösung auf den Träger eingesetzt werden.

Der Fachmann kann die Größe der BET-Oberfläche des Mischoxids durch bekannte Maßnahmen einstellen, um beispielsweise Oberflächen von kleiner als 155 m²/g zu erhalten. Je höher der Anteil an Siliciumdioxid im Mischoxid ausfällt, desto höher wird die Oberfläche sein. Demzufolge sind maximal 14 Gew.-% Siliciumdioxid, bezogen auf das Gesamtgewicht des Mischoxids, enthalten. Weiterhin beeinflusst die Calcinierungstemperatur die Oberfläche: Je niedriger die Temperatur eingestellt ist, desto höher wird die Oberfläche sein. Demzufolge liegt die Calcinierungstemperatur nicht unter 300 °C, vorzugsweise nicht unter 400 °C. Darüber hinaus sind vorzugsweise keine Polymere zur Vergrößerung der Oberfläche, wie dies beispielsweise in EP-A-2108631 (US 2009/0255402) beschrieben ist, enthalten. Durch wenige Versuche kann der Fachmann durch die genannten Parameter eine Einstellung der Oberfläche vornehmen.

Das Mischoxid kann vor oder nach Calcination in Formkörper umgeformt werden, wobei die Formkörperbildung vor der Calcination bevorzugt ist. Die formbare Masse kann organische Binder (z. B. Polymere wie Celluloseether, Polysaccharide, Polyethylenoxid), Porenbildner (z.B. Wachse, organische Polymere, vorzugsweise keine siliciumorganischen Verbindungen), anorganische Säuren wie Salpetersäure, anorganische Basen wie Natronlauge oder Mischungen daraus enthalten. Mit den Säuren bzw. Basen lassen sich die Festigkeit und Verformbarkeit des Mischoxids einstellen. Als Porenbildner für das System III vorzugsweise ausgenommen sind Polyalkylenpyrrolidonen wie Polyvinylpyrrolidon, Polyamine, Polyacrylate, Polyalkohole, Polysiloxane oder deren Mischungen, die die Bildung einer bimodalen Porenverteilung, wie beispielweise in EP-A-1074301 beschrieben, begünstigen. Beispiel eines solchen Polymers ist Poly(vinylpyrollidon) (PVP).

Einen weiteren Gegenstand der Erfindung bildet die Verwendung einer Katalysatorzusammensetzung K zur Herstellung von Ketonen aus einer mindestens eine Epoxidgruppe enthaltenden Verbindung. Die Katalysatorzusammensetzung K umfasst mindestens ein Edelmetall und mindestens ein Mischoxid, wobei das Mischoxid Zirconiumdioxid und Siliciumdioxid enthält und das Massenverhältnis von Zirconiumdioxid zu Siliciumdioxid im Mischoxid 86 : 14 bis 99,9 bis 0,1 beträgt. Das Edelmetall kann auf dem Mischoxid geträgert sein (System III).

Vorzugsweise wird eine erfindungsgemäße Katalysatorzusammensetzung bzw. eine nach einem erfindungsgemäßen Verfahren hergestellte Katalysatorzusammensetzung eingesetzt, wobei System II bevorzugt ist.

Ein weiterer Gegenstand der Erfindung ist ein Verfahren zur Herstellung von Ketonen, vorzugsweise Cyclododecanon, aus einer mindestens eine Epoxidgruppe enthaltenden Verbindung, wobei eine Katalysatorzusammensetzung K eingesetzt wird. Vorzugsweise wird eine erfindungsgemäße Katalysatorzusammensetzung bzw. eine nach einem erfindungsgemäßen Verfahren hergestellte Katalysatorzusammensetzung eingesetzt, wobei System II bevorzugt ist.

Das Verfahren stellt eine heterogene Katalyse dar.

Der Stoffmengenanteil an Edelmetall, bezogen auf die Stoffmenge der mindestens eine Epoxidgruppe enthaltenden Verbindung (Verbindung E), kann 0,00001 bis 0,1, vorzugsweise 0,0001 bis 0,01, betragen. Der Stoffmengenanteil an Mischoxid des Katalysatorsystems, bezogen auf die Stoffmenge der Verbindung E, kann 0,001 bis 100, vorzugsweise 0,005 bis 5, betragen.

Die Verbindung E kann aliphatisch oder cycloaliphatisch sein, wobei cycloaliphatische Verbindungen bevorzugt sind. Vorzugsweise sind 4 bis 20 C-Atome, bevorzugt 6 bis 16 C-Atome, besonders bevorzugt 8 bis 14 C-Atome, ganz besonders bevorzugt 10 bis 12 C-Atome und insbesondere 12 C-Atome umfasst.

Die Verbindung E kann eine oder mehrere Epoxid-Gruppen enthalten, wobei Mono-EpoxidVerbindungen bevorzugt sind.
Weiterhin kann die Verbindung gesättigt oder ungesättigt sein. Beispielsweise können eine oder zwei Doppelbindungen enthalten sein.

Bevorzugte Verbindungen E sind Monoepoxy-Cycloalkane, Monoepoxy-Cycloalkandiene und Monoepoxy-Cycloalkene, wobei Monoepoxy-Cycloalkane besonders bevorzugt sind. Eine ganz besonders bevorzugte Verbindung E ist Monoepoxy-cyclododecan.

Es hat sich gezeigt, dass die Bildung des entsprechenden Alkohol-Derivats als Nebenprodukt vom Druck des Wasserstoffs abhängt: Mit steigendem Druck erhöht sich der Alkohol-Anteil, so dass die Keton-Selektivität abnimmt.

Das erfindungsgemäße Verfahren kann bei einem Wasserstoffdruck von 0 bis zu 100 bar ausgeführt werden, wobei der Wasserstoffdruck vorzugsweise auf 0 bis 5 bar und bevorzugt auf 0 bis 2,5 bar eingestellt ist. Besonders bevorzugt beträgt der Wasserstoffdruck 0 bis 0,9 bar, ganz besonders bevorzugt 0 bis 0,5 bar. Das erfindungsgemäße Verfahren kann ohne Wasserstoff durchgeführt werden. Es ist jedoch zur Unterbindung ungesättigter Nebenprodukte bevorzugt, zumindest einen geringen Wasserstoffanteil vorzulegen. Dieser kann 0,05 bis 0,5 bar, vorzugsweise 0,1 bis 0,4 bar aufweisen. Alternativ kann ein Hydrierungsschritt nach der Umlagerung vorgesehen sein.

Die zuvor genannten Druckangaben beziehen sich auf den Partialdruck an Wasserstoff in dem System. Üblicherweise sind Komponenten des Reaktionsgemisches, einschließlich des Lösemittels, Luft oder Inertgase wie Stickstoff oder Argon weitere gasförmige Bestandteile des Systems.

Durch die niedrigen Wasserstoffdrücke ist gegenüber dem Stand der Technik ein deutlich geringerer technischer Aufwand insbesondere bei der geeigneten Apparatur notwendig, um mit Wasserstoff arbeiten zu können. Der besondere Vorteil der Erfindung liegt darin, dass das Keton in hohen Ausbeuten ohne die Anwesenheit von Wasserstoff gewonnen werden kann. Die Temperatur während der Reaktion wird vorzugsweise auf einen Bereich von 100 bis 350 °C, bevorzugt 150 bis 250 °C und besonders bevorzugt zwischen 180 und 230°C eingestellt. Die Reaktion kann durchgeführt werden mit einer Verbindung E, die sich im flüssigen oder gasförmigen Zustand befindet.

Das erfindungsgemäße Verfahren kann in organischen Lösemitteln durchgeführt werden, wobei es bevorzugt ist, ohne Lösemittel zu arbeiten und somit keine organischen Lösemittel einzusetzen. Geeignete Lösemittel sind beispielsweise Alkane wie n-Hexan, n-Heptan, n-Tetradecan und Cyclohexan; Ether wie Tetrahydrofuran und Dioxan; Alkanole wie Methanol, Ethanol und t-Butanol; Ester wie Ethylacetat und Butylacetat. Die Lösemittel können für sich allein oder in Mischungen eingesetzt werden. Das Lösemittel wird vorzugsweise in einer Menge eingesetzt, welche das 20-fache oder weniger, bevorzugt das 10-fache oder weniger des Gewichts der Verbindung E, beträgt.

Das Verfahren kann kontinuierlich oder diskontinuierlich ausgeführt werden. Die Aufreinigung des Ketons kann durch Destillation, Kristallisation oder Umkristallisation erfolgen.

In einer bevorzugten Ausführungsform der Erfindung wird Monoepoxy-cyclododecan zu Cyclododecanon ohne Lösemittel bei Temperaturen von 170 bis 250 °C umgesetzt, wobei als Katalysatorsystem eine Mischung von Palladium auf inertem Träger mit einem Palladium-Anteil von 0,5 bis 2 Gew.-%, bezogen auf das Gesamtgewicht des Trägers, und von einem Mischoxid aus der Calcination eines Gemisches umfassend mindestens Zirconiumhydroxid und Siliciumdioxid eingesetzt wird (System II). Bei der Reaktion werden maximal 0,9 bar Wasserstoff, ganz besonders bevorzugt maximal 0,1 bar eingesetzt.

Ein weiterer Gegenstand der Erfindung ist ein Verfahren zur Synthese von Lactamen (erfindungsgemäßes Lactamverfahren), bei dem das zuvor genannte erfindungsgemäße Verfahren zur Herstellung von Ketonen herangezogen wird. Die Verbindung E wird vorzugsweise ausgewählt aus aliphatischen Monoepoxy-Cycloalkane, aliphatischen Monoepoxy-Cycloalkandienen und aliphatischen Monoepoxy-Cycloalkenen, wobei Monoepoxy-Cycloalkane bevorzugt sind.

Sofern das Keton in einem Gemisch mit dem entsprechenden Alkohol-Derivat vorliegt, kann eine Dehydrierung des Alkohols zum Keton erfolgen. Anschließend kann das Keton oximiert werden. Im Folgeschritt kann die Beckmann-Umlagerung zum Lactam erfolgen, wobei die Umlagerung mittels Schwefelsäure oder Cyanurchlorid erfolgen kann. Die Lactame können unter Polykondensation zu Polyamiden weiterverarbeitet werden.

Die Dehydrierung, die Oximierung, die Beckmann-Umlagerung sowie die Kondensationsreaktion sind dem Fachmann bekannt.

In einer bevorzugten Ausführungsform des erfindungsgsgemäßen Lactamverfahrens wird aus Monoepoxy-cyclododecan (bzw. Cyclododecanepoxid oder 1,2-Cyclododecanepoxid) Laurinlactam hergestellt.

Im Rahmen des bevorzugten Lactamverfahrens kann Monoepoxy-cyclododecan gewonnen werden durch folgende Reaktionsschritte: 1,3-Butadien wird durch Cyclotrimerisation zu Cyclododecatrien umgesetzt. Anschließend erfolgt eine Hydrierung zum Cyclododecen. Durch nachfolgende Epoxidierung wird das Cyclododecanepoxid erhalten. Der Fachmann auf dem Gebiet der Synthese organischer Verbindungen kann andere aliphatische und cycloaliphatische Verbindungen E in Analogie zu der Synthese von Monoepoxy-cyclododecan herstellen.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keinesfalls als in irgendeiner Weise limitierende Offenbarung aufzufassen.

Nachfolgend wird die vorliegende Erfindung anhand von Beispielen näher erläutert. Alternative Ausführungsformen der vorliegenden Erfindung sind in analoger Weise erhältlich.

### Beispiele

Die prozentuale Angabe bei Katalysatoren gibt den Gewichtsanteil des Edelmetalls an, bezogen auf das Gesamtgewicht des Katalysators umfassend Edelmetall und Träger. Die Abkürzung "calc." steht für "calciniert". Die Abkürzungen der Stoffe sind: CDAN: Cyclododecan; CDEN: Cyclododecen; ECD: Epoxycyclododecan; CDON: Cyclododecanon; CDENON: Cyclododecenon (Isomerenmischung); CDOL: Cyclododecanol; CDENOL: Cyclododecenol (Isomerenmischung).

Die Gewichtsangaben des Edelmetalls beziehen sich, soweit nichts anderes angegeben ist, auf das Gesamtgewicht des Trägers des Edelmetalls.

Das in den Beispielen eingesetzte Siliciumdioxid (Aerosil) wurde über ein Pyrogenverfahren erhalten.

### Herstellung der Mischoxide

### Beispiel A (nicht erfindungsgemäß): Herstellung von ZrO₂ Formkörpern

Oxid 1 (100% ZrO₂): 2000 g Zirconiumhydroxidpulver (XZO 1501/09, Fa. MEL Chemicals) wurden in einem Muffelofen bei 450°C für 3 h zu Zirconiumdioxid calciniert. Nach Abkühlung wurden 1000 g Zirconiumdioxidpulver mit 10 g Celluloseether (Tylose MH1000 P2 der Fa. SE Tylose) gemischt. Anschließend wurden 520 g Wasser zugegeben, und die Mischung wurde so lange geknetet, bis eine extrudierbare Masse erhalten wurde. Die Masse wurde mittels eines Doppelwalzenextruders zu Strängen mit einem Durchmesser von 1,6 mm verarbeitet und zerschnitten. Die Grünkörper wurden 4 Stunden bei 110 °C getrocknet und anschließend 2 Stunden bei 450 °C calciniert.

Die BET Oberfläche der calcinierten Formkörper betrug 64 m²/g. Die Porenradienverteilung war monomodal.

Das Oxid 1 entspricht dem Zirconiumdioxid, das in Tabelle 3, letzter Eintrag, und Tabelle 4, vierter und fünfter Eintrag, der EP-A-2772478 eingesetzt wurde.

### Beispiel B: Herstellung von Formkörpern aus ZrO₂ und SiO₂ (Mischoxid)

Oxid 2 (95 % ZrO₂, 5 % SiO₂): 773 g Zirconiumdioxidpulver (aus Beispiel A) wurden mit 40 g Siliciumdioxid (Aerosil 200 V, Fa. Evonik), und 8 g Tylose MH1000 gemischt. Anschließend wurden 450 g Wasser zugegeben, und die Mischung wurde so lange geknetet, bis eine extrudierbare Masse erhalten wurde. Die Masse wurde mittels eines Doppelwalzenextruders zu Strängen mit einem Durchmesser von 1,6 mm verarbeitet und zerschnitten. Die Grünkörper wurden 4 Stunden bei 110 °C getrocknet und anschließend 2 Stunden bei 450°C calciniert.

Die Porenradienverteilung war monomodal.

Oxid 3 (95 % ZrO₂, 5 % SiO₂): 1000 g Zirconiumhydroxid (XZO 1501/09, Fa. MEL Chemicals), 40 g Aerosil 200 V, 20 g Wachs (Licowax C Micro Powder PM der Fa. Clariant) und 2 g Celluloseether (Tylose MH1000 P2 der Fa. SE Tylose) wurden in einem Mischer vorgelegt und trocken gemischt. Danach wurden 8,3 g einer 30%igen NaOH-Lösung in 800 g Wasser zugegeben und die Mischung wurde so lange geknetet, bis eine extrudierbare Masse erhalten wurde. Die Masse wurde mittels eines Doppelwalzenextruders zu Strängen mit einem Durchmesser von 1,8 mm verarbeitet und zerschnitten. Die Grünkörper wurden 4 Stunden bei 110 °C getrocknet und anschließend 2 Stunden bei 450 °C calciniert.

Die calcinierten Formkörper enthielten ZrO₂ und SiO₂ in einem Massenverhältnis von 95:5. Die calcinierten Formkörper hatten einen mittleren Durchmesser von 1,16 mm und eine BET-Oberfläche von 150 m²/g.

Weitere Oxide (4 bis 9) wurden unter analogen Bedingungen hergestellt. Variiert wurde die Menge an Zirconiumhydroxid, Siliciumdioxid sowie Art und Menge der Zirconiumverbindungen B und anorganischer Säuren bzw. Basen. Als pulvriges Formhilfsmittel wurden immer 20 g Licowax und 2 g Tylose MH1000 P2 zugegeben. Die genauen Bedingungen sind in der folgenden Tabelle erfasst.

In den Oxiden 10 und 11 wurden Ammoniumzirconiumcarbonat (Bacote 20, Oxid 10) bzw. Zirconylnitrat (Oxid 11) als Zirconiumverbindung B eingesetzt.

### Beispiel C (nicht erfindungsgemäß): Herstellung von ZrO₂-dotiertem SiO₂

Oxid 12 (15 % ZrO₂, 85 % SiO₂): 85 g SiO₂-Träger (Aerolyst® 3041, Fa. Evonik) wurden mit 85 mL einer 5,5 Gew% NaOH Lösung imprägniert und bei 110 °C für 4 h getrocknet. Parallel wurde 40 g ZrOCl₂·8H₂O in Wasser gelöst und die Lösung bis 85 mL verdünnt. Der getrocknete Träger wurde mit der Zirkonylchlorid-Lösung imprägniert, bei 110 °C über Nacht getrocknet und anschließend bei 450°C für 2 Stunden calciniert.

Oxid 13 (15 % ZrO₂, 85 % SiO₂): 50 g einer 30%-Zirkonacetatlösung (Zr(OAc)₄) wurde bis 85 mL verdünnt. 85 g SiO₂-Träger (Aerolyst® 3041, Fa. Evonik) wurde mit der Zirkonylchlorid-Lösung imprägniert, bei 110 °C über Nacht getrocknet und anschließend bei 450°C für 2 Stunden calciniert.

Die Porenradienverteilungen sämtlicher Träger der Beispiele A bis C waren monomodal.

**Tabelle 1. Übersicht der hergestellten Mischoxide**

| Oxid | Zr-Präkursor | SiO₂ | Sonstiges | ZrO₂:SiO₂ (Massenverhältnis) | BET (m²/g) |
|---|---|---|---|---|---|
| 3 | 1000 g Zr(OH)₄ | 40 g Aerosil 200V | 8,3 g einer 30%igen NaOH Lösung in 800 g H₂O | 95:5 | 150 |
| 4 | 1000 g Zr(OH)₄ | 8 g Aerosil 200V | 65,4 g einer 65%igen HNO₃ Lösung in 530 g H₂O | 99:1 | 78 |
| 5 | 1000 g Zr(OH)₄ | 24 g Aerosil 200V | 66 g einer 65%igen HNO₃ Lösung in 550 g H₂O | 97:3 | 79 |
| 6 | 1000 g Zr(OH)₄ | 40 g Aerosil 200V | 84 g einer 65%igen HNO₃ Lösung in 541 g H₂O | 95:5 | 86 |
| 7 | 1000 g Zr(OH)₄ | 85,5 g Aerosil 200V | 84 g einer 65%igen HNO₃ Lösung in 565 g H₂O | 90:10 | 109 |
| 8* | 894 g Zr(OH)₄ | 122 g Aerosil 200V | 7,45 g einer 30%igen NaOH Lösung in 830 g H₂O | 85:15 | 213 |
| 9* | 1000 g Zr(OH)₄ | 142 g Tetraethylorthosilicat | 400 g Wasser | 95:5 | --- |
| 10 | 1000 g Zr(OH)₄ | 40 g Aerosil 200 V | 8,3 g einer 30%igen NaOH Lösung in 700 g H₂O, 118 g Bacote 20 (Fa. MEL Chemicals) | 95:5 | 131 |
| 11 | 1000 g Zr(OH)₄ | 40 g Aerosil 200 V | 90 g einer 65%igen HNO₃ Lösung in 450 g H₂O, 118 g Zirconylnitrat (Fa. MEL Chemicals) | 95:5 | 113 |
| 12* | 40 g ZrOCl₂· 8H₂O | 85 g Silica-Träger | 85 mL einer 5,5 Gew.% NaOH Lösung | 15:85 | --- |
| 13* | 50 g Zr(OAc)₄ (30 %ig) | 85 g Silica-Träger | --- | 15:85 | --- |

| | | | | | |
|---|---|---|---|---|---|
| * *nicht erfindungsgemäß* | | | | | |

### Katalysatorzusammensetzungen

### Bespiel D: Zusammensetzung von Mischoxid und Pd-imprägniertem Siliziumdioxid (Katalysatorsystem II)

2000 g Siliciumdioxid-Formkörper (Aerolyst® 3041, Fa. Evonik) wurden in eine rotierende Glastrommel eingefüllt und bis 110°C erhitzt. Parallel wurden 100 g einer 10%igen Pd(II)-Nitrat-Lösung gewogen und durch Zugabe von Wasser auf 1910 g verdünnt. Die Lösung wurde dann auf den SiO₂-Träger gesprüht. Es entstand eine dünne Edelmetall-Schale. Die getränkten Formkörper wurden anschließend 10 Stunden in einer reduzierenden Atmosphäre (0,4 Vol.-% Wasserstoff im Stickstoff) bei 200 °C calciniert. Der Gewichtsanteil an Pd bezüglich des SiO₂-Trägers betrug 0,5 %. Diese Pd/SiO₂ Formkörper wurden mit den verschiedenen Oxiden 1 bis 13 aus den Beispielen A, B, C zusammengesetzt.

### Katalysatorzusammensetzungen:

- Zusammensetzung 1*: Oxid 1 (50 Gew.-%) und Pd/SiO₂ (50 Gew.-%); diese Zusammensetzung enthält die gleichen Bestandteile wie die Katalysatorzusammensetzungen, die in Tabelle 3, letzter Eintrag, und Tabelle 4, vierter und fünfter Eintrag, der EP-A-2772478 eingesetzt wurden
- Zusammensetzung 2: Oxid 2 (50 Gew.-%) und Pd/SiO₂ (50 Gew.-%)
- Zusammensetzung 3: Oxid 3 (50 Gew.-%) und Pd/SiO₂ (50 Gew.-%)
- Zusammensetzung 4: Oxid 4 (50 Gew.-%) und Pd/SiO₂ (50 Gew.-%)
- Zusammensetzung 5: Oxid 5 (50 Gew.-%) und Pd/SiO₂ (50 Gew.-%)
- Zusammensetzung 6: Oxid 6 (50 Gew.-%) und Pd/SiO₂ (50 Gew.-%)
- Zusammensetzung 7: Oxid 7 (50 Gew.-%) und Pd/SiO₂ (50 Gew.-%)
- Zusammensetzung 8*: Oxid 8 (50 Gew.-%) und Pd/SiO₂ (50 Gew.-%)
- Zusammensetzung 9*: Oxid 9 (50 Gew.-%) und Pd/SiO₂ (50 Gew.-%)
- Zusammensetzung 10: Oxid 10 (23 Gew.-%) und Pd/SiO₂ (77 Gew.-%)
- Zusammensetzung 11: Oxid 11 (23 Gew.-%) und Pd/SiO₂ (77 Gew.-%)
- Zusammensetzung 12: Oxid 3 (9 Gew.-%) und Pd/SiO₂ (91 Gew.-%)
- Zusammensetzung 13: Oxid 3 (23 Gew.-%) und Pd/SiO₂ (77 Gew.-%)
- Zusammensetzung 14 : Oxid 3 (33 Gew.-%) und Pd/SiO₂ (67 Gew.-%)
- Zusammensetzung 15 : Oxid 6 (20 Gew.-%) und Pd/SiO₂ (80 Gew.-%)
- Zusammensetzung 16*: TiO₂-SiO₂ Mischung (calciniert) *VP TiO2 545 S* aus Pyrogenverfahren von Evonik Industries (9 Gew.-%) und Pd/SiO₂ (91 Gew.-%)
- Zusammensetzung 17*: TiO₂-SiO₂ Mischung (calciniert) *VP TiO2 590 S* aus Pyrogenverfahren von Evonik Industries (9 Gew.-%) und Pd/SiO₂ (91 Gew.-%)
- Zusammensetzung 18*: SiO₂ Aerosil® 200V (50 Gew.-%) und Pd/SiO₂ (50 Gew.-%)
- Zusammensetzung 19*: Oxid 12 (50 Gew.-%) und Pd/SiO₂ (50 Gew.-%)
- Zusammensetzung 20*: Oxid 13 (50 Gew.-%) und Pd/SiO₂ (50 Gew.-%)
- Zusammensetzung 21*: Oxid 1 (33,3 Gew.-%) und Pd/SiO₂ (67,7 Gew.-%); diese Zusammensetzung entspricht der Katalysatorzusammensetzung, die in Tabelle 3, letzter Eintrag, der EP-A-2772478 eingesetzt wurde
- Zusammensetzung 22*: Oxid 1 (9 Gew.-%) und Pd/SiO₂ (91 Gew.-%); diese Zusammensetzung enthält die gleichen Bestandteile wie die Katalysatorzusammensetzungen, die in Tabelle 3, letzter Eintrag, und Tabelle 4, vierter und fünfter Eintrag, der EP-A-2772478 eingesetzt wurden
** nicht erfindungsgemäß*

Zusammensetzungen 1, 21 und 22 umfassen kein Mischoxid im Sinne der Erfindung; sie stellenphysikalische Mischungen aus Zirconiumdioxid und Siliciumdioxid dar (SiO₂ als Träger).

### Beispiel E: Pd auf Mischoxid geträgert (Katalysatorsystem III)

75 g Oxidformkörper gemäß Beispiel C wurden in einem Polybeutel gewogen. Parallel wurden 3,75 g einer 10%igen Pd(II)-Nitrat-Lösung gewogen und durch Zugabe von Wasser auf 45 mL verdünnt. Die verdünnte Lösung wurde auf die Formkörper in den Polybeutel gegossen, und der Beutel wurde immer wieder geschüttelt, bis die Flüssigkeit homogen auf den Formkörpern verteilt war. Die getränkten Formkörper wurden 2,5 Stunden bei 110 °C getrocknet, und anschließend 10 Stunden in einer reduzierenden Atmosphäre (0,4 Vol.-% Wasserstoff im Stickstoff) bei 200 °C calciniert. Der Gewichtsanteil an Pd im Katalysator, bezogen auf das Gesamtgewicht des Mischoxids, betrug 0,5 %.

### Katalysatorzusammensetzungen:

- Zusammensetzung 23*: Oxid 1 mit 0,5 Gew.-% Pd geträgert
- Zusammensetzung 24*: Oxid 1 mit 0,5 Gew.-% Pd geträgert (95 Gew.-%), nach der Imprägnierung und Calcination gemischt mit SiO₂ Aerosil® 200V (5 Gew.-%). Hier liegt eine physikalische Mischung von ZrO₂ und SiO₂ vor, kein Mischoxid.
- Zusammensetzung 25: Oxid 3 mit 0,5 Gew.-% Pd

### * nicht erfindungsgemäß

### Katalysatortestung

Gaschromatographie (GC): Gaschromatographische Untersuchungen erfolgten mit einem GC-2010 (Shimadzu) Chromatographen, ausgestattet mit Autosampler,
Flammenionisationsdetektor (FID) sowie GC-Kapillarsäule Supelcowax® (60 m × 0.32 mm × 0.25 µm, Supelco). Messungen wurden im Split-Modus (Split-Rate 1 : 66) mit Helium als Trägergas (Flussrate 0,89 mL/min, lineare Trägergasgeschwindigkeit 17,9 cm/sec) durchgeführt. Temperaturprogramm für GC-Ofen: Starttemperatur 150 °C; mit 5 °C/min bis auf 180 °C heizen, für 10 min halten; mit 5 °C/min bis auf 200 °C heizen, für 10 min halten. Detektor- und Injektortemperaturen betrugen 340 und 220 °C.

### Beispiel 1: Vergleich verschiedener Oxide (Katalysatorsystem II)

Die Reaktion wurde in einem 500 mL-Rundkolben mit mechanischer Rührung durchgeführt. Die Beheizung des Reaktors erfolgte mit einem elektrischen Aluminiumheizblock und die Innentemperatur wurde mit einem Thermofühler kontrolliert. Der Kolben wurde mit 50 mL 1,2-Cyclododecanepoxid, 20 g Katalysatorzusammensetzungen aus Beispiel D gefüllt. Das Katalysatorbett lag am Boden des Kolbens und das flüssige Reaktionsgemisch wurde über dem Bett gerührt. Der Kolben wurde anschließend mit Stickstoff inertisiert und bis 215 °C Innentemperatur erhitzt. Die Temperatur wurde während 1,5 Stunden gehalten.

**Tabelle 2. Zusammensetzung (Flächen-%, GC) der Reaktionsmischung (nach 1,5 h)**

| **Katalysatorzusammensetzung** | **Umsatz (%)** | **Selektivität (%)** | | | | |
|---|---|---|---|---|---|---|
| | | **CDAN** | **CDEN** | **CDON + CDENON** | **CDOL** | **CDENOL** |
| *1** | 28 | 0 | 0 | 74 | 6 | 18 |
| (physikalische Mischung) | | | | | | |
| *2* | 64 | 0,6 | 0 | 79 | 6 | 14 |
| *(ZrO₂-SiO₂ 95*/*5)* | | | | | | |
| *4* | 55 | 0 | 0 | 90 | 5 | 5 |
| *(ZrO₂-SiO₂ 99*/*1)* | | | | | | |
| *5* | 71 | 0 | 0,6 | 81 | 9 | 10 |
| *(ZrO₂-SiO₂ 97*/*3)* | | | | | | |
| *3* | 97 | 0,4 | 3 | 79 | 6 | 11 |
| *(ZrO₂-SiO₂ 95*/*5)* | | | | | | |
| *6* | 80 | 0,1 | 1,5 | 79 | 7 | 10 |
| *(ZrO₂-SiO₂ 95*/*5)* | | | | | | |
| *7* | 86 | 0,1 | 4,4 | 69 | 9 | 17 |
| *(ZrO₂-SiO₂ 90*/*10)* | | | | | | |
| *8** | 95 | 0 | 10 | 66 | 6 | 17 |
| *(ZrO₂-SiO₂ 85*/*15, BET>200)* | | | | | | |
| *9** | 77 | 0 | 2 | 16 | 1 | 72 |
| *(Organo-Si Quelle)* | | | | | | |
| *16** | 51 | 1 | 1 | 3 | 0,5 | 60 |
| *(TiO₂-SiO₂ 95*/*5)* | | | | | | |
| *17** | 60 | 0,9 | 2 | 7 | 0,7 | 63 |
| *(TiO₂-SiO₂ 95*/*5)* | | | | | | |
| *18** | < 1 | Nicht relevant | | | | |
| *(SiO₂)* | | | | | | |
| *19** | 86 | 0,3 | 9 | 70 | 9 | 11 |
| *(ZrO₂-SiO₂ 15*/*85)* | | | | | | |
| *20** | >99 | < 1 % CDONgebildet, (siehe Tabelle 4) | | | | |
| *(ZrO₂-SiO₂ 15*/*85)* | | | | | | |

| | | | | | | |
|---|---|---|---|---|---|---|
| ** nicht erfindungsgemäß* | | | | | | |

In Tabelle 2 sind die Selektivitäten für CDON und CDENON zusammengefasst dargestellt, da diese technisch relevant sind. Hintergrund ist, dass CDENON üblicherweise durch den Zusatz von Wasserstoff in CDON umgesetzt wird. Insofern stellt CDENON kein Nebenprodukt dar. Dennoch wird in der folgenden Tabelle 3 der Vollständigkeit halber zwischen CDON und CDENON differenziert.

**Tabelle 3. Zusammensetzung (Flächen-%, GC) der Reaktionsmischung (nach 1,5 h) gemäß Tabelle 2; Aufteilung der Mischung CDON + CDENON**

| **Katalysatorzusammensetzung** | **Umsatz (%)** | **Selektivität (%)** | | |
|---|---|---|---|---|
| | | CDON | CDENON | **CDON + CDENON** |
| *1** | 28 | 68 | 6 | 74 |
| (physikalische Mischung) | | | | |
| *2* | 64 | 72 | 7 | 79 |
| *(ZrO₂-SiO₂ 95*/*5)* | | | | |
| *4* | 55 | 84 | 6 | 90 |
| *(ZrO₂-SiO₂ 99*/*1)* | | | | |
| *5* | 71 | 74 | 7 | 81 |
| *(ZrO₂-SiO₂ 97*/*3)* | | | | |
| *3* | 97 | 69 | 10 | 79 |
| *(ZrO₂-SiO₂ 95*/*5)* | | | | |
| *6* | 80 | 69 | 10 | 79 |
| *(ZrO₂-SiO₂ 95*/*5)* | | | | |
| *7* | 86 | 54 | 15 | 69 |
| *(ZrO₂-SiO₂ 90*/*10)* | | | | |
| *8** | 95 | 52 | 14 | 66 |
| *(ZrO₂-SiO₂ 85*/*15*, *BET>200)* | | | | |
| *9** | 77 | 12 | 4 | 16 |
| *(Organo-Si Quelle)* | | | | |
| *16** | 51 | 0 | 3 | 3 |
| (*TiO₂*-*SiO₂ 95*/*5)* | | | | |
| *17** | 60 | 0 | 7 | 7 |
| *(TiO₂-SiO₂ 95*/*5)* | | | | |
| *18** | < 1 | | Nicht relevant | |
| *(SiO₂)* | | | | |
| *19** | 86 | 53 | 17 | 70 |
| *(ZrO₂-SiO₂ 15*/*85*) | | | | |
| *20** | >99 | < 1 % CDON gebildet (siehe Tabelle 4) | | |
| *(ZrO₂-SiO₂ 15*/*85)* | | | | |

| | | | | |
|---|---|---|---|---|
| ** nicht erfindungsgemäß* | | | | |

Mit der gleichen Reaktionszeit war der Umsatz mit der Zusammensetzung 1 deutlich geringer als mit den Zusammensetzungen 2 bis 7. Es zeigte sich, dass die Mischoxide ZrO₂-SiO₂ bei ähnlicher Selektivität zum Keton eine viel höhere katalytische Aktivität als reines ZrO₂ aus dem Stand der Technik haben. Die Aktivität stieg mit dem Anteil an SiO₂ im Mischoxid an.

Mit der Zusammensetzung 8 wurden 10 % des Nebenprodukt CDEN erhalten, so dass dieser Katalysator wegen der geringen Selektivität nicht wirtschaftlich eingesetzt werden kann. Ein SiO₂-Anteil von > 15 Gew.-% im Mischoxid und eine BET-Oberfläche > 200 m²/g sind deswegen nicht bevorzugt.

Die Zusammensetzungen 9 (Organosilicium-Quelle) sowie 16 und 17 (chemische Mischung aus TiO₂ und SiO₂) zeigen eine sehr geringe Selektivität zu CDON.

Die Zusammensetzung 18 hat keine katalytische Aktivität. Dieser Versuch bestätigt, dass ZrO₂ im Katalysatorsystem einen zwingend notwendigen Bestandteil bildet.

Die Zusammensetzung 19 (Zirconiumdioxid-dotierte Siliziumoxide) zeigt relativ hohe Umsätze und hohe Selektivitäten. Allerdings berücksichtigen die in den Tabellen 2 und 3 dargestellten Daten nicht die Bildung von Hochsiedern (Oligomere und Polymere), die bei der Reaktion entstehen. In der folgenden Tabelle 4 werden die Werte für die Hochsieder berücksichtigt.

**Tabelle 4. Zusammensetzung (Gew-%, mit GC-Faktoren berechnet) einiger Reaktionsmischungen nach 5 h unter Berücksichtigung der Hochsieder**

| **Katalysatorzusammensetzung** | **CDAN + CDEN** | **Epoxid** | **CDON** | **CDENON** | **CDOL** | **CDENOL** | **Hochsieder (Oligomere/ Polymere)** |
|---|---|---|---|---|---|---|---|
| *1** | 0,3 | 42 | 51 | 3 | 2,6 | 1,7 | 0,3 |
| (physikalische Mischung) | | | | | | | |
| *3* | 2,7 | 0 | 79 | 9 | 5 | 1,4 | 2,6 |
| *(ZrO₂-SiO₂ 95*/*5)* | | | | | | | |
| *19** | 9 | 0 | 54 | 12 | 5 | 2,4 | 17 |
| *(ZrO₂-SiO₂ 15*/*85)* | | | | | | | |
| *20** | 1 | 0,4 | 0,5 | 9 | 0,25 | 4 | 85 |
| *(ZrO₂-SiO₂ 15*/*85)* | | | | | | | |

| | | | | | | | |
|---|---|---|---|---|---|---|---|
| ** nicht erfindungsgemäß* | | | | | | | |

Mit den Zusammensetzungen 19 und 20, jeweils Zirkonium-dotiertes Siliciumdioxid, werden Anteile von 17 % bzw. 85 % an Hochsiedern erhalten. Die erfindungsgemäße Zusammensetzung umfassend das Mischoxid hingegen weist lediglich 2,6 % Hochsieder als Nebenprodukt auf. Ein mit Zirconiumdioxid dotiertes oder beschichtetes Siliziumdioxid ist also als Katalysatorsystem nicht geeignet.

### Beispiel 2: Vergleich verschiedener Oxide (Katalysatorsystem II) - zeitlicher Verlauf

Die oben genannte Reaktion des Beispiels 1 wurde für die Zusammensetzungen 1 und 3 fortgesetzt bis zu einer Reaktionszeit von 5 h. Es wurden regelmäßig Proben entnommen und die Umsetzung des Edukts 1,2-Cyclododecanepoxid bestimmt. Darüber hinaus wurde ein weiterer Versuch mit 30 g der Zusammensetzung 21 durchgeführt.

**Tabelle 5. Umsatz des Edukts 1,2-Cyclododecanepoxid über einen Zeitraum von 5 h (Flächen-%, GC)**

| **Katalysatorzusammensetzung** | **Symbol in** **Fig. 1** | **Umsatz Edukt** | | | | |
|---|---|---|---|---|---|---|
| | | **0 h** | **0,5 h** | **1,5 h** | **3,0 h** | **5,0 h** |
| *1** | Raute | 0 % | 5 % | 28 % | 46 % | 58 % |
| *(physikalische Mischung)* | | | | | | |
| 3 | Quadrat | 0 % | 67 % | 97 % | 100 % | 100 % |
| *(ZrO₂-siO₂ 95*/*5)* | | | | | | |
| 21* | Dreieck | 0 % | 40 % | 80 % | 94 % | 100 % |
| *(physikalische Mischung)* | | | | | | |

| | | | | | | |
|---|---|---|---|---|---|---|
| ** nicht erfindungsgemäß* | | | | | | |

Die erfindungsgemäße Zusammensetzung 3 führt bereits nach 1,5 h zu einer nahezu vollständigen Umsetzung des eingesetzten Edukts, der nach 3 h 100 % erreicht. Die Mischung 1 erreicht diesen Wert selbst nach 5 h Reaktionszeit nicht. Die Mischung 21 des Standes der Technik (EP-A-2772478, Tabelle 3, letzter Eintrag) zeigt zwar ebenfalls einen vollständigen Umsatz; dieser liegt allerdings erst nach deutlich längerer Reaktionszeit vor und wird mit einer höheren Menge an Katalysatorzusammensetzung erreicht.

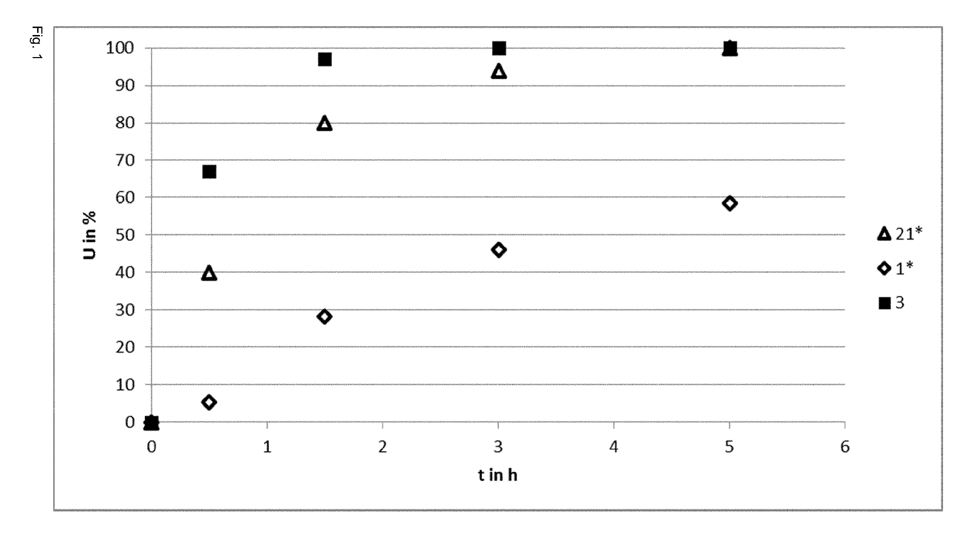

Der Verlauf der Zusammensetzung ist in Figur 1 graphisch dargestellt. Dabei wurde die Umsetzung U an 1,2-Cyclododecan-Epoxid (in Prozent) gegen die Reaktionszeit t in Stunden aufgetragen.

### Beispiel 3: Edelmetall auf dem Mischoxid geträgert (Katalysatorsystem III)

Die Reaktion wurde in einem 500 mL-Rundkolben mit mechanischer Rührung durchgeführt. Die Beheizung des Reaktors erfolgte mit einem elektrischen Aluminiumheizblock und die Innentemperatur wurde mit einem Thermofühler kontrolliert. Der Kolben wurde mit 50 mL 1,2-Cyclododecanepoxid und der Katalysatorzusammensetzung (Beispiel E) gefüllt. Das Katalysatorbett lag am Boden des Kolbens und das flüssige Reaktionsgemisch wurde über dem Bett gerührt. Der Kolben wurde anschließend mit Stickstoff inertisiert und bis 215 °C Innentemperatur erhitzt. Die Temperatur wurde während 1,5 Stunden gehalten.

**Tabelle 6. Zusammensetzung (Flächen-%, GC) der Reaktionsmischung (nach 1,5 h)**

| **Katalysatorzusammensetzung** | **Menge (g)** | **Umsatz (%)** | **Selektivität (%)** | | | | |
|---|---|---|---|---|---|---|---|
| | | | **CDAN** | **CDEN** | **CDON** | **CDOL** | **CDENOL** |
| *23** | 10 | 35 | 0 | 0 | 94 | 4 | 0 |
| *(ZrO₂)* | | | | | | | |
| *25* | 10 | 98 | 0,3 | 1,1 | 79 | 8 | 3 |
| *(ZrO₂-SiO₂ 95*/*5)* | | | | | | | |
| *23** | 20 | 80 | 1 | 0 | 96 | 3 | 0 |
| *(ZrO₂)* | | | | | | | |
| *24** | 20 | 69 | 1 | 0 | 97 | 2 | 0 |
| *(physikalische Mischung)* | | | | | | | |
| *25* | 20 | >99 | 0,5 | 1,4 | 87 | 5 | 2 |
| *(ZrO₂-SiO₂ 95*/*5)* | | | | | | | |

| | | | | | | | |
|---|---|---|---|---|---|---|---|
| ** nicht erfindungsgemäß* | | | | | | | |

Beispiel 3 hat demonstriert, dass ZrO₂-SiO₂-Mischoxide des Katalysatorsystems III eine katalytische Aktivität aufwiesen. Diese fiel für das Mischoxid (Zusammensetzung 25) deutlich höher aus als für Katalysatoren ohne SiO₂ (Zusammensetzung 23) oder Katalysatoren umfassend eine physikalische Mischung aus ZrO₂ und SiO₂ (Zusammensetzung 24).

### Beispiel 4: Optimierte Reaktionsbedingungen (Katalysatorsystem II)

Die Reaktion wurde in einem 500 mL Rundkolben mit mechanischer Rührung durchgeführt. Die Beheizung des Reaktors erfolgte mit einem elektrischen Aluminiumheizblock und die Innentemperatur wurde mit einem Thermofühler kontrolliert. Der Kolben wurde mit 50 mL 1,2-Cyclododecanepoxid und 13 g der Katalysatorzusammensetzung gefüllt. Das Katalysatorbett lag am Boden des Kolbens und das flüssige Reaktionsgemisch wurde über dem Bett gerührt. Der Kolben wurde anschließend mit Stickstoff inertisiert und bis 215 °C Innentemperatur erhitzt. Die Temperatur wurde während 6 Stunden gehalten.

Das Gewichtsverhältnis von ZrO₂ zu SiO₂ betrug bei allen drei Katalysatorzusamensetzungen 95:5. Ebenso war das Gewichtsverhältnis von Oxid zu Pd auf einem inerten Träger identisch (23:67). Die Oxide wurden entweder aus Zirconiumhydroxid oder aus Zirconiumhydroxid und einer Verbindung B hergestellt.

**Tabelle 7. Zusammensetzung (Flächen-%, GC) der Reaktionsmischung (nach 6 h)**

| **Katalysatorzusammensetzung** | **Umsatz (%)** | **Selektivität (%)** | | | | |
|---|---|---|---|---|---|---|
| | | **CDAN** | **CDEN** | **CDON + CDENON** | **CDOL** | **CDENOL** |
| *10* | 98 | 1 | 0,4 | 87 | 5 | 4 |
| *(Ammoniumzirconium-carbonat)* | | | | | | |
| *11* | 67 | 0,1 | 1,3 | 88 | 6 | 4 |
| *(Zirconylnitrat)* | | | | | | |
| *13* | 98 | 0,1 | 1,2 | 91 | 4 | 1,3 |
| *(keine Verbindung B)* | | | | | | |

Eine zweite Zirconiumverbindung B, zusätzlich zum Zirconiumhydroxid A, wurde in der Herstellung der Mischoxide 10 und 11 zugegeben. Die Zusammensetzung 13 wurde mit Zirconiumhydroxid A als einzige Zr-Quelle hergestellt. Die Katalysatorzusammensetzungen sind somit unabhängig von der Zusammensetzung der Zirconiumverbindung für die Umlagerung des Epoxids zum Keton geeignet.

Analog zu Tabelle 7 werden in Tabelle 8 die Anteile an CDON und CDENON einzeln aufgeführt.

**Tabelle 8. Zusammensetzung (Flächen-%, GC) der Reaktionsmischung (nach 1,5 h) gemäß Tabelle 7; Aufteilung der Mischung CDON + CDENON**

| **Katalysatorzusammensetzung** | **Umsatz (%)** | **Selektivität (%)** | | |
|---|---|---|---|---|
| | | **CDON** | **CDENON** | **CDON + CDENON** |
| *10* | 98 | 79 | 8 | 87 |
| *(Ammoniumzirconiumcarbonat)* | | | | |
| *11* | 67 | 79 | 9 | 88 |
| *(Zirconylnitrat)* | | | | |
| *13* | 98 | 84 | 7 | 91 |
| *(keine Verbindung B)* | | | | |

### Beispiel 5: Optimierte Reaktionsbedingungen (Katalysatorsystem II)

Die Reaktion wurde in einem 500 mL-Rundkolben mit mechanischer Rührung durchgeführt. Die Beheizung des Reaktors erfolgte mit einem elektrischen Aluminiumheizblock und die Innentemperatur wurde mit einem Thermofühler kontrolliert. Der Kolben wurde mit 50 mL 1,2-Cyclododecanepoxid, und 10 g 0,5 Gew.-% Palladium auf Siliciumdioxid als Formkörper und dem Mischoxid des Katalysatorsystems (Formkörper) gefüllt. Das Katalysatorbett lag am Boden des Kolbens und das flüssige Reaktionsgemisch wurde über dem Bett gerührt. Der Kolben wurde anschließend mit Stickstoff inertisiert und 180 bis 215 °C Innentemperatur erhitzt. Die Temperatur wurde während der gegebenen Zeit gehalten.

**Tabelle 9. Zusammensetzung (Flächen-%, GC) der Reaktionsmischung**

| **Katalysatorzusammensetzung** | **Mischoxid** | **Temp (°C)** | **Zeit (h)** | **Umsatz (%)** | **Selektivität (%)** | | | |
|---|---|---|---|---|---|---|---|---|
| | | | | | **CDAN** | **CDEN** | **CDON + CDENON** | **CDOL** |
| 1* | *10 g Oxid 1* | 215 | 5 | 58 | 0 | 0,2 | 92 | 4 |
| 22* | *1 g Oxid 1* | 215 | 5 | 13 | 0 | 0 | 95 | 4 |
| 12 | *1 g Oxid 3* | 215 | 5 | 50 | 0,4 | 1 | 92 | 3 |
| 1* | *10 g Oxid 1* | 215 | 24 | 98 | 0 | 0 | 97 | 2 |
| 22* | *1 g Oxid 1* | 215 | 24 | 38 | 0 | 0 | 96 | 3 |
| 12 | *1 g Oxid 3* | 215 | 24 | 98 | 0 | 1,5 | 94 | 4 |
| 13 | *3g Oxid 3* | 200 | 24 | 99 | 0,2 | 0,5 | 95 | 3 |
| 1* | *10 g Oxid 1* | 180 | 24 | 19 | 0 | 0 | 94 | 3 |
| 3 | *10 g Oxid 3* | 180 | 24 | >99 | 0 | 0,7 | 93 | 5 |

| | | | | | | | | |
|---|---|---|---|---|---|---|---|---|
| ** nicht erfindungsgemäß* | | | | | | | | |

**Tabelle 10. Zusammensetzung (Flächen-%, GC) der Reaktionsmischung gemäß Tabelle 9; Aufteilung der Mischung CDON + CDENON**

| **Katalysatorzusammensetzung** | **Mischoxid** | **Temp (°C)** | **Zeit (h)** | **Umsatz (%)** | **Selektivität (%)** | | |
|---|---|---|---|---|---|---|---|
| | | | | | **CDON** | **CDENON** | **CDON + CDENON** |
| 1* | *10 g Oxid 1* | 215 | 5 | 58 | 87 | 5 | 92 |
| 22* | *1 g Oxid 1* | 215 | 5 | 13 | 94 | 1 | 95 |
| 12 | *1 g Oxid 3* | 215 | 5 | 50 | 75 | 17 | 92 |
| 1* | *10 g Oxid 1* | 215 | 24 | 98 | 95 | 2 | 97 |
| 22* | *1 g Oxid 1* | 215 | 24 | 38 | 95 | 1 | 96 |
| 12 | *1 g Oxid 3* | 215 | 24 | 98 | 87 | 7 | 94 |
| 13 | *3 g Oxid 3* | 200 | 24 | 99 | 90 | 5 | 95 |
| 1* | *10 g Oxid 1* | 180 | 24 | 19 | 92 | 2 | 94 |
| 3 | *10 g Oxid 3* | 180 | 24 | >99 | 86 | 7 | 93 |

| | | | | | | | |
|---|---|---|---|---|---|---|---|
| ** nicht erfindungsgemäß* | | | | | | | |

Ein Vergleich der erfindungsgemäßen Zusammensetzung 12 (ZrO₂-SiO₂-Mischoxid) mit den nicht-erfindungsgemäßen Zusammensetzungen 1 und 22 (Stand der Technik) während einer Reaktionszeit von 5 h bei 215 °C belegt, dass weniger als 1/10 an ZrO₂ für die gleiche katalytische Aktivität benötigt wird. Mit der gleichen Menge an Zirconiumdioxid zeigt die erfindungsgemäße Zusammensetzung 12 eine viel höhere katalytische Aktivität als die Zusammensetzung 22 (Stand der Technik).

Der 24-stündige Versuch demonstriert, dass für einen ähnlich hohen Umsatz bei gleichzeitig ähnlich hoher Selektivität im Vergleich zur Zusammensetzung 1 weniger Katalysator (Zusammensetzungen 12, 13) oder eine geringere Temperatur benötigt werden (Zusammensetzungen 13, 3).

### Beispiel 6: Umsetzung von Epoxid

Die Reaktion wurde in einem 500 mL Rundkolben mit mechanischer Rührung durchgeführt. Die Beheizung des Reaktors erfolgte mit einem elektrischen Aluminiumheizblock und die Innentemperatur wurde mit einem Thermofühler kontrolliert. Der Kolben wurde mit 50 mL 1,2-Cyclododecanepoxid, 10 g 0,5 Gew.-% Palladium auf Siliciumdioxid als Formkörper und 5 g Oxid 3 gefüllt (entspricht 15 g der Zusammensetzung 14). Das Katalysatorbett lag am Boden des Kolbens und das flüssige Reaktionsgemisch wurde über dem Bett gerührt. Es wurde eine Mischung aus Wasserstoff und Stickstoff während der Reaktion durchgeleitet (1 bar mit 90 Vol.-% H₂ und 10 Vol.-% N₂). Der Kolben wurde anschließend bis 215 °C Innentemperatur erhitzt. Die Temperatur wurde während 5 Stunden gehalten. Am Ende des Versuchs wurde eine Mischung von CDON (87,2 %), CDOL (8,4 %), CDAN (2 %) und CDENOL (1 %) erhalten.

### Beispiel 7: Festbettverfahren

Die Reaktion wurde in einer Festbettanlage durchgeführt. Die Anlage bestand aus zwei Festbettreaktoren in Serie (ca. 200 mL pro Reaktor) und einem Vorlagestahlbehälter (1 L). Der untere Festbettreaktor wurde mit 50 g Oxid 6 und der obere Festbettreaktor wurde mit 200 g von 0,5% Pd/SiO₂ aus Beispiel D befüllt. Diese Zusammensetzung entspricht 250 g der Katalysatorzusammensetzung 15. Der Behälter wurde mit 1500 g 1,2-Cyclododecanepoxid befüllt. Die Flüssigkeit wurde im Kreis aus der Vorlage von unten nach oben durch das Katalysatorbett zurück in den Vorlagebehälter mittels einer Umwälzpumpe (10 l/h) gepumpt. Die Reaktoren wurden bis 205 °C im Reaktionsgemisch mit einer elektrischen Heizung aufgeheizt. Das Reaktionsgemisch wurde mit Stickstoff durch die Festbettreaktoren und den Behälter überleitet. Nach 30 h Reaktion wurde ein Umsatz von 78 % mit einer Selektivität von ca. 93 % CDON erreicht.

**Tabelle 11. Zusammensetzung (Flächen-%, GC) der Reaktionsmischung**

| **Temp (°C)** | **Zeit (h)** | **Umsatz (%)** | **Selektivität (%)** | | | | | |
|---|---|---|---|---|---|---|---|---|
| | | | **CDAN** | **CDEN** | **CDON** | **CDENON** | **CDOL** | **CDENOL** |
| 205 | 30 | 78 | 0,4 | 1 | 93 | 0,9 | 4 | 0,4 |

Die Reaktion kann mit den erfindungsgemäßen Katalysatorsystemen in Festbettreaktoren durchgeführt werden. Dieses Experiment zeigt weiterhin, dass beide Komponenten des Katalysatorsystems II (ZrO₂-SiO₂-Mischoxid als Formkörper und Pd/SiO₂-Formkörper) in räumlich getrennten Reaktoren ohne negative Auswirkung auf die Aktivität und Selektivität eingesetzt werden können.

## Patentansprüche

1. Katalysatorzusammensetzung umfassend mindestens ein Edelmetall und mindestens ein Mischoxid, wobei das Mischoxid Zirconiumdioxid und Siliciumdioxid umfasst, **dadurch gekennzeichnet, dass** das Edelmetall geträgert ist und der Träger nicht aus dem Mischoxid besteht, und wobei das Massenverhältnis von Zirconiumdioxid zu Siliciumdioxid im Mischoxid 86 : 14 bis 99,9 : 0,1 beträgt.

2. Katalysatorzusammensetzung nach Anspruch 1, **dadurch gekennzeichnet, dass** der Träger nicht das Mischoxid enthält.

3. Katalysatorzusammensetzung nach einem der vorherigen Ansprüche, **dadurch gekennzeichnet, dass** der Träger des Edelmetalls ausgewählt ist aus Siliciumdioxid, Aluminiumoxid oder Mischungen daraus.

4. Katalysatorzusammensetzung nach einem der vorherigen Ansprüche, **dadurch gekennzeichnet, dass** das Edelmetall ausgewählt ist aus Ruthenium, Rhodium, Palladium, Osmium, Iridium, Platin und Mischungen daraus, vorzugsweise Palladium.

5. Verfahren zur Herstellung einer Katalysatorzusammensetzung nach einem der vorherigen Ansprüche, **dadurch gekennzeichnet, dass** zur Herstellung des Mischoxids
a) eine formbare Masse hergestellt wird, welche zumindest umfasst
i. eine Zirconiumverbindung,
ii. Siliciumdioxid als Feststoff, vorzugsweise mittels Pyrogenverfahren hergestellt, und
iii. Wasser, und
b) die formbare Masse bei einer Temperatur von 300 bis 500 °C calciniert wird.

6. Verfahren nach Anspruch 5, **dadurch gekennzeichnet, dass** das Edelmetall zur Herstellung des Katalysatorsystems auf einen inerten Träger imprägniert wird.

7. Verfahren nach einem der Ansprüche 5 oder 6, **dadurch gekennzeichnet, dass** die Zirconiumverbindung ausgewählt ist aus Zirconiumdioxid, Zirconiumhydroxid, Zirconiumacetat, Zirconiumnitrat, Zirconiumoxychlorid, Ammoniumzirconiumcarbonat oder Mischungen daraus, bevorzugt Zicroniumdioxid, Zirconiumhydroxid oder Mischungen daraus.

8. Verfahren nach Anspruch 7, **dadurch gekennzeichnet, dass** die Zirconiumverbindung eine Mischung aus A und B darstellt, wobei
A ausgewählt ist aus Zirconiumdioxid, Zirconiumhydroxid und Mischungen daraus und
B ausgewählt ist aus Zirconiumacetat, Zirconiumnitrat, Zirconiumoxychlorid, Ammoniumzirconiumcarbonat und Mischungen daraus.

9. Verfahren nach einem der Ansprüche 5 bis 8, **dadurch gekennzeichnet, dass** das Siliciumdioxid als Feststoff eine Korngröße d₅₀ von 100 nm bis 500 µm (Laserbeugung) aufweist.

10. Verfahren nach einem der Ansprüche 5 bis 9, **dadurch gekennzeichnet, dass** die formbare Masse mindestens einen Stoff enthält, der ausgewählt ist aus der Gruppe der organischen Binder, Porenbildner, anorganischen Säuren, anorganischen Basen und Mischungen daraus.

11. Verfahren nach einem der Ansprüche 5 bis 10, **dadurch gekennzeichnet, dass** die formbare Masse vor der Calcinierung zu Formkörpern umgeformt wird.

12. Verwendung einer Katalysatorzusammensetzung der Ansprüche 1 bis 4 zur Herstellung von Ketonen aus einer mindestens eine Epoxidgruppe enthaltenden Verbindung.

13. Verfahren zur Herstellung von Ketonen, vorzugsweise Cyclododecanon, aus einer mindestens eine Epoxidgruppe enthaltenden Verbindung, wobei eine Katalysatorzusammensetzung der Ansprüche 1 bis 4 eingesetzt wird.

14. Verfahren zur Synthese von Lactamen, bei dem ein Verfahren zur Herstellung von Ketonen nach Anspruch 13 herangezogen wird.
